# EUROPEAN PATENT APPLICATION

(11) **EP 4 729 628 A1**
(43) Date of publication of application: **22.04.2026**
(21) Application number: 24206660.3
(22) Date of filing: 15.10.2024
(51) Int. Cl.: C12Q 1/6839

(54) **LABEL, MARKER AND METHOD FOR ANALYSING A BIOLOGICAL SAMPLE**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

In a first aspect, a label (100, 300, 500, 702, 802, 900, 901, 1000, 1200, 1300) for analysing a biological sample is provided. The label comprises a nucleic acid backbone (102, 304, 502, 1002, 1102, 1202, 1301) and a first plurality of labelling moieties (104). The nucleic acid backbone (102, 304) comprises a triplex structure (306a, 306b, 306c). In further aspects a marker (700, 800) comprising the label and a method for analysing biological samples is provided.

## Description

### Technical field

The invention relates to a label for analysing a biological sample, the label comprising a nucleic acid backbone with a triplex structure. In further aspects, a marker comprising the label and a method for analysing a biological sample with the marker are provided.

### Background

The use of markers with labels that comprise a large number of individual dyes finds frequent application in fields like molecular biology and diagnostics, offering unparalleled sensitivity and specificity in detecting biological entities and chemical processes, in particular with fluorescence microscopy. These markers enable the visualization of complex cellular structures, tracking of molecular interactions, and high-throughput assays with high resolution. In particular, labels with a large number of dyes may result in bright labels.

However, the incorporation of numerous individual dyes within markers introduces a set of challenges, most notably self-quenching. Self-quenching occurs when the proximity of dyes within a marker leads to non-radiative energy transfer among them, significantly diminishing the emission efficiency and the fluorescence intensity that can be detected. This reduction in signal not only affects the sensitivity of measurements but also complicates quantitative analysis, making it difficult to accurately determine the concentration of targets or the efficiency of binding events. Self-quenching further poses a considerable challenge in high-resolution studies.

### Summary

It is an object to provide a label and a marker that are bright and have efficient emission characteristics.

The aforementioned object is achieved by the subject-matter of the independent claims. Advantageous embodiments are defined in the dependent claims and the following description.

In a first aspect, a label for analysing a biological sample is provided. The label comprises a nucleic acid backbone and a first plurality of labelling moieties. The nucleic acid backbone comprises a triplex structure, in particular a plurality of triplex structures.

In particular, a triplex structure is a segment or section of the nucleic acid backbone that comprises three single stranded nucleic acid strands, which are hybridised to each other to form a triple helix. The three single stranded nucleic acid strands do not necessarily have to be separate, individual nucleic acid molecules, rather at least some of the three single stranded nucleic acid strands may be a continuous nucleic acid molecule that hybridises to itself.

The label enables bright emission of the plurality of labelling moieties, in particular with native optical properties of the labelling moieties, at a high efficiency. The triplex structure of the nucleic acid backbone provides a rigid or stiff backbone to attach the labelling moieties to. The rigidity or stiffness enables keeping the labelling moieties in place relative to each other and avoiding an aggregation of the labelling moieties, for example due to an attractive force between labelling moieties. Aggregation of labelling moieties may cause a change of their native optical properties such as their emission intensity and/or absorbance spectra. This makes unambiguous identification of the labelling moieties difficult or impossible.

A measure of the degree of rigidity or stiffness of a nucleic acid backbone, in particular a triplex structure is persistence length (L_{P}). The persistence length is a mechanical parameter quantifying polymer rigidity: the higher the persistence length, the more rigid the polymer. For example, in the presence of monovalent or divalent salts, a nucleic acid duplex structure regularly has a persistence length of 30 to 55 nm, while single stranded nucleic acid is much more flexible, with a persistence length of 1.5 to 3 nm.

Thus, a persistence length of the nucleic acid backbone in the range of 30 to 55 nm is for example achieved by using a double-stranded DNA backbone. To further decrease the flexibility of said nucleic acid backbone (and increase persistence length), the nucleic acid backbone is configured such that it can form at least one triplex with a third DNA strand or oligonucleotide. Thus, the nucleic acid backbone of the label may preferably have a persistence length greater than 30 to 55 nm. This enables generating a rigid nucleic acid backbone as a stable framework for attaching labelling moieties. The rigidity of the nucleic acid backbone further enables bright fluorescent emission of the attached labelling moieties with their native optical properties.

The persistence length may be measured or determined using various methods including but not limited to single-molecule stretching techniques (e.g. optical, magnetic tweezers, dynamic force spectroscopy), FRET, dynamical mean-field theory and fluorescence correlation spectroscopy, atomic force microscopy as described in Roth et al. Nano Lett. 2018, 18, 11, 6703-6709, who used DNA-origami rods connected by a stretch of ssDNA of varying length and AFM to measure the persistence length in a way that allows the impact of secondary structures to be evaluated as well.

Qingjia Chi, Guixue Wang and Jiahuan Jiang (The persistence length and length per base of single-stranded DNA obtained from fluorescence correlation spectroscopy measurements using mean field theory; in Physica A: Statistical Mechanics and its Applications, Volume 392, Issue 5, 2013, Pages 1072-1079) discuss persistence length in relation to nucleic acids.

The labelling moieties, particularly of the first plurality of labelling moieties, are preferably optically detectable. For example, the labelling moieties may be fluorescent. Thus, the labelling moieties may be fluorophores, such as a fluorescent protein, an organic or inorganic fluorescent molecule, or a fluorescent nanoparticle, which may be optically detectable e.g. with a fluorescence microscope. Preferably, the first plurality of labelling moieties comprises between 2 and 200 labelling moieties, more preferably between 10 and 150 labelling moieties. In particular, each labelling moiety is attached to the nucleic acid backbone, preferably covalently attached. Alternatively, each labelling moiety may be attached to the nucleic acid backbone by a high affinity interaction such as a biotin-streptavidin linker.

Preferably, a brightness of the label, in particular of the labelling moieties, in the presence of the triplex structure is at least 10% higher than a label without the triplex structure.

Preferably, adjacent labelling moieties of the first plurality of labelling moieties are attached to the nucleic acid backbone at a distance from each other between 1 to 50 nm. In a particularly preferred embodiment, adjacent labelling moieties of the first plurality of labelling moieties are attached to the nucleic acid backbone at a distance from each other of 5 to 40 nucleotides corresponding to about 1 to 13.2 nm, or 8 to 20 nucleotides corresponding to about 2.6 to 6.7nm. This enables dense spacing of the labelling moieties whilst avoiding electronic interactions between labelling moieties.

In some embodiments, where a label comprises a first and second labelling moiety such as fluorescent dyes of a FRET pair, it may be desirable to have a very dense packing of a large number of FRET donors that function like an antenna to collect excitation light and funnel the excitation to a smaller number, or may be even just one, FRET acceptor.

In some embodiments multiple labels collective comprising a large number of FRET donors, such as 10, 50, 100, 1,000 FRET donors, being arranged using DNA nanotechnology to collect light and transfer energy to a small number of or a single FRET acceptor.

Preferably, the triplex structure comprises at least 10%, more preferably at least 20%, of nucleotides of the nucleic acid backbone. Thus, at least 10% of the nucleotides of the nucleic acid backbone may be comprised in the segment or section of the nucleic acid backbone that forms the triplex structure. In a particular embodiment, essentially the entire nucleic acid backbone is a triplex structure. This provides a particular robust and rigid nucleic acid backbone.

Preferably, the nucleic acid backbone comprises a main oligonucleotide with at least a first nucleotide sequence, wherein the nucleic acid backbone comprises at least a second nucleotide sequence, and wherein at least the first nucleotide sequence and the second nucleotide sequence form a duplex structure. Thus, the nucleic acid backbone may comprise the duplex structure.

Preferably, the first nucleotide sequence and the second nucleotide sequence are at least partially complementary to each other, in particular based on Watson-Crick base pairing. Thus, the first and the second sequence may form the duplex structure with each other. This enables stable hybridisation of the first nucleotide sequence and the second nucleotide sequence to each other. The duplex may be a double helix, such as B-form DNA.

Thus, the duplex structure may be based on complementary base-pairing, for example, of at least one continuous sequence of nucleotides, in particular the main oligonucleotide, of the nucleic acid backbone. For example, the duplex structure may be a secondary or tertiary structure, such as a double helix. In a particular example, the nucleic acid backbone may comprise several discontinuous double stranded segments that are the duplex structure of the nucleic acid backbone.

Preferably, the nucleic acid backbone comprises at least a third oligonucleotide with a third nucleotide sequence, and wherein the third nucleotide sequence forms the triplex structure with the first nucleotide sequence and the second nucleotide sequence.

Preferably, the third nucleotide sequence is at least partially complementary to the hybridised first nucleotide sequence and second nucleotide sequence, in particular based on Hoogsteen base-pairing.

Thus, the third nucleotide sequence can hybridise to the duplex of the first nucleic acid sequence and the second nucleic acid sequence, in particular to a major groove of the duplex. This enables a stable triplex. In particular, the third nucleic acid sequence has a respective sequence that is at least partially complementary to the duplex of the first nucleic acid sequence and the second nucleic acid sequence.

In particular, additional sections of the nucleic acid backbone may have a duplex structure, to which the third nucleotide sequence does not hybridise. In a particular embodiment, the entire nucleic acid backbone may be a duplex structure, to only a section of which the third nucleotide sequence hybridises to form the triplex structure.

Preferably, the nucleic acid backbone comprises several third oligonucleotides. Each third oligonucleotide may have a third nucleic acid sequence that may be complementary to a different part of the duplex of the first and second nucleotide sequences. In particular, the label may comprise between 3 and 5 third oligonucleotides. Thus, the third oligonucleotide may hybridise to different sections of the nucleic acid backbone, each third oligonucleotide forming a triplex along the different sections with the first and second sequences.

Preferably, the main oligonucleotide further comprises the second nucleotide sequence, and wherein the second nucleotide sequence is a reverse complement of the first nucleotide sequence. This enables generating the nucleic acid backbone from few individual oligonucleotides and, in particular, generating a robust label. Thus, the complementary first and second nucleotide sequences form intramolecular base-pairs. For example, the nucleic acid backbone may form a stem-loop or hairpin loop, wherein the stem forms part of the duplex structure.

Preferably, the nucleic acid backbone comprises several of the main oligonucleotide. This enables generating a bright label, for example, comprising a large number of labelling moieties. The several main oligonucleotides may be connected to each other, for example by means of complementary sequences on each main oligonucleotide, or by means of connecting oligonucleotides that are complementary sequences of each main oligonucleotide. For example, each of the several main oligonucleotides may form a stem-loop or hairpin loop. In particular, each of the several main oligonucleotides may comprise a first and second nucleotide sequence that are unique to the particular one of the several main oligonucleotides. Thus, the first and the second nucleotide sequence of one of the several main oligonucleotides may only hybridise to each other and not to other first or second nucleotide sequences of other main oligonucleotides. Further, each of the main oligonucleotides may be hybridised to at least one respective third oligonucleotide to form the triplex structure.

Preferably, the nucleic acid backbone comprises at least one auxiliary oligonucleotide comprising the second nucleotide sequence. This enables flexible generation of the duplex structure of the nucleic acid backbone of the label by addition of the at least one auxiliary oligonucleotide. Thus, in contrast to the embodiment where the first and second sequences are comprised by the main oligonucleotide, the first and second sequences may be on separate oligonucleotides. Thus, the main and auxiliary oligonucleotides may be separate oligonucleotides. In case the nucleic acid backbone comprises several auxiliary oligonucleotides, the main oligonucleotide may comprise several first nucleotide sequences and each auxiliary oligonucleotide may comprise the second nucleotide sequence. In particular, each second nucleotide sequence may be unique to a particular one of the auxiliary oligonucleotides and therefore hybridise to a particular one of the complementary first nucleotide sequences.

Preferably, the main oligonucleotide and the auxiliary oligonucleotide are cross-linked with each other. This enables a particularly robust label. Further, the cross-link is preferably a covalent bond, for example comprising a triazole compound. The crosslinkage may be generated by click chemistry, such as copper-catalysed azide-alkyne cycloaddition. For example, the main oligonucleotide and/or the at least one auxiliary oligonucleotide may comprise a first reaction moiety and a second reaction moiety, respectively. When generating the label, initially the oligonucleotides of the nucleic acid backbone may be hybridised based on the complementary first and second sequences, subsequently, the oligonucleotides may be cross-linked in order to stabilise the label. Generally, the cross-linkages increase the rigidity or stiffness of the nucleic acid backbone, in particular of the duplex structure.

Alternatively or in addition, in case the nucleic acid backbone comprises several auxiliary oligonucleotides, the several auxiliary oligonucleotides may preferably be cross-linked with each other. In particular, each auxiliary oligonucleotide may be cross-linked with auxiliary oligonucleotides that are adjacent along the complementary main oligonucleotide.

Alternatively or in addition, the main oligonucleotide may preferably be cross-linked with itself. For example, a part of the main oligonucleotide towards a first end of the main oligonucleotide may be cross-linked to a part of the main oligonucleotide towards a second end of the main oligonucleotide. This enables generating a rigid circular label. In particular, the circular shape may generate ring tension, which significantly reduces conformational flexibility of the nucleic acid backbone.

Preferably, the nucleic acid backbone comprises a plurality of the first nucleotide sequence. This enables generating a label with a complex duplex structure. For example, the nucleic acid backbone may comprise a stem-loop formed based on one first and second nucleotide sequence and the nucleic acid backbone may comprise another first nucleotide sequence for hybridising to an auxiliary oligonucleotide.

Preferably, the label comprises at least one nucleic acid binding molecule. This enables increasing the rigidity or stiffness of the nucleic acid backbone of the label. In particular, by aligning or ordering the nucleic acid backbone around the nucleic acid binding molecule. Examples of nucleic acid binding molecules include (eukaryotic) histones and bacterial DNA binding proteins, such as HU or integration host factor (IHF). A particular example of a bacterial DNA binding protein is Bd0055.

The nucleic acid binding molecule preferably binds to the triplex structure and/or duplex structure of the nucleic acid backbone. For example, the bacterial DNA binding protein Bd0055 binds duplex DNA on its outside surface and induces a rod formation in the duplex/triplex DNA. The binding of the nucleic acid binding molecules may be non-specific or sequence specific. In the latter case, the nucleic acid backbone, in particular the triplex structure, may comprise specific sequences configured to bind nucleic acid binding molecules.

Preferably, the labelling moieties are hydrophobic labelling moieties. The label, in particular its nucleic acid backbone, provides a rigid structure for hydrophobic labelling moieties. Generally, in an aqueous solution hydrophobic labelling moieties have a tendency to aggregate. In particular, hydrophobic labelling moieties that are arranged in close proximity on a flexible single stranded DNA backbone may lead to formation of aggregates. This aggregation changes the optical properties of these labelling moieties such as their absorption wavelength and/or in particular their fluorescence brightness, compared to non-aggregated hydrophobic labelling moieties. By providing the label with the hydrophobic labelling moieties, the nucleic acid backbone avoids the aggregation of the labelling moieties and therefore enables use of the hydrophobic labelling moieties at their native optical properties.

Examples of hydrophobic labelling moieties that are regularly used to analyse biological samples include ATTO 390, ATTO 425, ATTO 550, ATTO Rho12, ATTO 633, and ATTO 647N. In particular, the dyes mentioned above may exhibit undesired aggregation when multimerized on a single stranded DNA nucleic acid backbone with a dye-to-dye distance of 8 nt.

In contrast, hydrophilic labelling moieties may include ATTO 488, and ATTO 532, and ATTO 565 for example. In particular, the dyes mentioned above do not exhibit undesired aggregation when multimerized on a ssDNA nucleic acid backbone with a dye-to-dye distance of 8 nt.

Preferably, the labelling moieties are attached to the nucleic acid backbone at a distance from each other in a range from one to ten nucleotides. This enables generating compact labels. Preferably, the labelling moieties are attached to nucleic acid backbone such that the labelling moieties are oriented in the same direction. In this case, the labelling moieties may be attached to every tenth nucleotide, in particular, of one of the oligonucleotides of the nucleic acid backbone.

Preferably, the labelling moieties are attached to the main oligonucleotide, in particular to the duplex structure or the triplex structure of the nucleic acid backbone. By attaching the labelling moieties to the duplex or triplex structure, the labelling moieties are attached to the part of the nucleic acid backbone that has an increased rigidity. This enables generating labels with labelling moieties, wherein the labelling moieties have a consistent distance from each other, that is not substantially influenced by the flexibility of the nucleic acid backbone. This avoids undesired interactions between the labelling moieties. In particular, the labelling moieties may be attached to the first nucleotide sequence or the second nucleotide sequence. In a particular embodiment, the labelling moieties are attached to the third nucleotide sequence.

Preferably, the auxiliary oligonucleotide or the third oligonucleotide comprises at least a second plurality of labelling moieties configured to excitonically interact, for example via a fluorescence resonance energy transfer, with the first plurality of labelling moieties, e.g. as described in WO 2023/198291 A1, the complete content thereof being incorporated by reference. This enables specifically influencing or modulating the optical properties of the first plurality of labelling moieties. In particular, this enables generating a large number of distinguishable labels.

Preferably, the label comprises a plurality of reactive oxygen species quenchers, wherein each of the reactive oxygen species quenchers is attached to the nucleic acid backbone in close proximity to a labelling moiety, in particular of the first plurality of labelling moieties. This enables an increase in the lifetime of the labelling moieties, in particular, by reducing the occurrence or concentration of reactive oxygen species in the proximity of the labelling moieties. This reduces the deleterious effects of reactive oxygen species on the labelling moieties. In particular, the close proximity of the reactive oxygen species quenchers to the labelling moieties may be chosen such that the quenching action of the quenchers has an advantageous effect on the labelling moieties. Preferably, the reactive oxygen species quenchers may be attached to the nucleic acid backbone at a distance in a range of 0.3 nm to 15 nm to one of the labelling moieties. Examples of reactive oxygen species quenchers include DABCO and lycopene. In a particular example, the labelling moieties may be attached to one of the main oligonucleotide, the auxiliary oligonucleotide, and the third oligonucleotide and the reactive oxygen species quencher may be attached to the another one of the main oligonucleotide, the auxiliary oligonucleotide, and the third oligonucleotide.

Preferably, the nucleic acid backbone comprises 20 to 260 nucleotides, and/or wherein the main oligonucleotide has a length of 20 to 120 nucleotides, more preferably 100 to 120 nucleotides. The third oligonucleotide preferably comprises 6 to 120 nucleotides, more preferably 9 to 60, most preferably 9 to 22 nucleotides. The label may, in particular, comprise 3 to 5 third oligonucleotides. This enables generating a compact label.

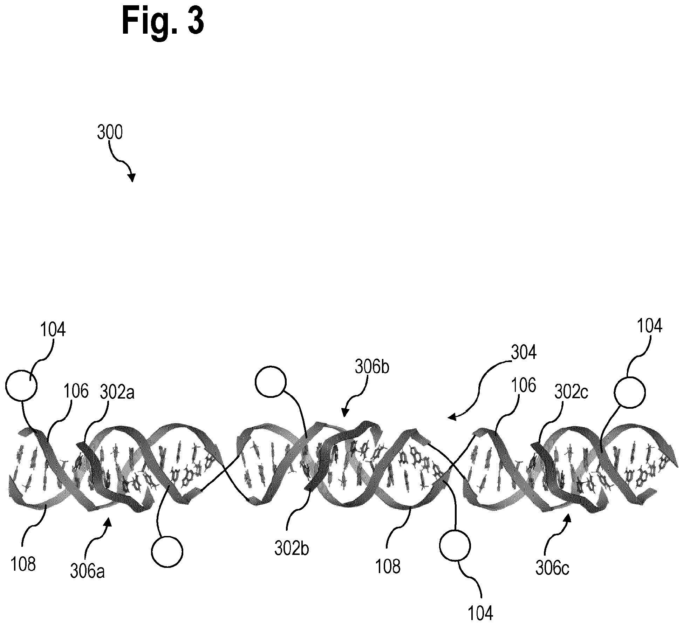

The nucleic acid backbone may comprise a single strand configured to fold-back on itself, i.e. forming a hairpin, like for example the following sequences published in Kumar et al., in particular in Figure 3: rHRP1, dHRP1, rHRP2, dHRP2. The third oligonucleotides may for example be the following sequences provided in Kumar et al., in particular in Figure 3: PNA1, PNA2, PNA3α,β, PNA4, PNASε, PNA6ε. As described in Kumar et al. "Triplex-Forming Peptide Nucleic Acids with Extended Backbones" Chembiochem. 2020 December 01; 21(23): 3410-3416 modified PNA.

Of particular interest in this regard are also the following PNA and DNA sequences studied by Hansen ME, Bentin T, Nielsen PE. (High-affinity triplex targeting of double stranded DNA using chemically modified peptide nucleic acid oligomers. Nucleic Acids Res. 2009 Jul;37(13):4498-507. doi: 10.1093/nar/gkp437. Epub 2009 May 27. PMID: 19474349; PMCID: PMC2715256). PNA sequences: 84, 1846, 2998, 2999, 3000, 3001, 3002, 3003, 3004, 3005, 3006, 3007, 3015, 3049, 3050, 3051, 3095, 3096, 3097; DNA sequences: TFO1, p322, p395, p396, p397. For these sequences the authors published EC50 values that range from µM to nM. A nucleic acid backbone may therefore comprise multiple copies of the sequences p322, p395, p396, p397, or a mix of such sequences. PNA1846-dsDNA, PNA3004-dsDNA, PNA3052-dsDNA triplexes have EC50 values in the range of <2nM to 7nM and may thus be well suited to generate labels according to the present disclosure.

Preferably, the nucleic acid backbone, particularly the main oligonucleotide, comprises a barcode sequence. The barcode sequence may be for hybridising the label to a complementary sequence of an affinity reagent, for example. This enables easily and specifically attaching the label to an affinity reagent. In particular, the barcode sequence may be comprised by the main oligonucleotide, for example as a single stranded free end of the main oligonucleotide.

Preferably, the label comprises at least 5 labelling moieties, in particular, the labelling moieties are covalently conjugated to one of the main oligonucleotide, the auxiliary oligonucleotide, and the third oligonucleotide.

In particular, the nucleic acid backbone may comprise or essentially consist of nucleic acid analogues, in particular modified nucleobases. Generally, nucleic acid analogues are compounds which are structurally similar to naturally occurring RNA and DNA. Nucleic acids are chains of nucleotides, which are composed of three parts: a phosphate backbone, a pentose sugar, either ribose or deoxyribose, and one of four nucleobases. An analogue may have any of these parts altered. Thus, a nucleic acid analogue may be a non-naturally occurring, modified, artificial, or xeno nucleic acid (XNA), in particular. Whereas natural nucleic acids or naturally occurring nucleic acids (DNA and RNA) are generally sensitive to natural degradation agents such as nucleases, nucleic acid analogues are generally resistant to these degradation agents such as nucleases. Thus, this enables providing a nucleic acid backbone or label resistant to degradation by natural degradation agents, such as nucleases, and therefore a particular robust label.

More specifically, nucleic acid analogues may comprise modified nucleobases or may comprise partially or entirely a xeno nucleic acid (XNA) like for example PT-DNA, L-DNA, LNA, morpholino, peptide nucleic acid (PNA). The use of XNA may help to improve nuclease-resistance, which is desired in some applications.

Alternatively, the nucleic acid backbone may comprise or essentially consists of natural occurring nucleic acids.

In another aspect, a marker for analysing a biological sample is provided. The marker comprises a label, in particular as described above. The marker further comprises an affinity reagent configured to specifically bind to a target analyte. The affinity reagent may be an antibody, an antibody fragment, or an aptamer, in particular a nucleic acid based aptamer, for example. The affinity reagent enables specifically binding the label to a particular target analyte in the biological sample.

Preferably, the affinity reagent comprises a barcode sequence. The barcode sequence is for hybridising the affinity reagent to a complementary barcode sequence of the label, for example. This enables easily and specifically attaching the label to the affinity reagent. Alternatively, the label may be attached to the affinity reagent by means of a covalent linker.

In a further aspect, a method for analysing a biological sample is provided. The method comprises the steps of introducing into the biological sample at least one marker and/or parts of at least one marker, in particular as described above. The method further includes the step of generating a readout of the biological sample with the marker. This enables identifying and/or locating the target analyte in the biological sample that the marker binds to. In particular, the readout may be an optical readout, for example, generated by means of a microscope.

In particular, the step of introducing into the biological sample parts of at least one marker includes introducing at least an affinity reagent, a nucleic acid backbone and a first plurality of labelling moieties into the biological sample. The parts may be introduced either separately from each other in time and/or spatially, or together. Thus, the marker may either be generated prior to introduction into the biological sample or the marker may be generated from its parts within the biological sample. The latter may be advantageous in case the marker, in particular the label, is bulky and/or rigid compared to its individual parts and the biological sample is a tissue section, for example. In this case, the more compact and/or flexible individual parts may be introduced separately and the bulky and/or rigid marker is only generated in situ. In particular, the formation of the duplex structure of the nucleic acid backbone of the label in situ may be advantageous, since the main oligonucleotide as a single strand may more easily penetrate or diffuse into the biological sample compared to the duplex/triplex structure. Thus, preferably the duplex/triplex structure of the nucleic acid backbone of the label may be generated in situ, for example by adding the auxiliary oligonucleotide and/or the third oligonucleotide after adding the main oligonucleotide of the nucleic acid backbone of the label.

In addition, the cross-linking of the main oligonucleotide and/or the auxiliary oligonucleotide may be carried out after introducing the parts of the marker into the biological sample.

Preferably, prior to or after generating the readout and after the step of introducing the parts of at least one marker, an environmental parameter is changed in order to generate or ungenerate the duplex structure. This enables controlling the fluorescent emission of the label. For example, the duplex/triplex structure of the nucleic acid backbone of the label may be melted by an increase in temperature, in particular above the melting temperature of the duplex/triplex structure, causing the nucleic acid backbone to relax and the labelling moieties to aggregate. Aggregation of labelling moieties may cause a change in fluorescent emission due to self-quenching. An optical readout might be generated in this situation. The duplex/triplex structure may be generated by subsequently reducing the temperature, causing the nucleic acid backbone to stiffen and the labelling moieties to separate. Preferably, labels with nucleic acid backbones comprising between 10 to 40 nucleotides are used when the method comprises steps of environmental parameters changes.

Generally, a plurality of markers may be introduced into the biological sample, the markers being specific to respective target analytes, in order to identify a large number of (different or the same) target analytes at the same time. Preferably, a target analyte is identified and/or localised within the biological sample based on the label(s) of the marker(s), in particular the labelling moieties, associated with the target analyte in the optical readout. Markers may be physically constituted before introducing them into the biological sample. Alternatively, affinity reagents may be barcoded with oligonucleotide barcodes and introduced into the biological sample initially and the labels comprising complementary barcode oligonucleotides may be introduced into the biological sample in a subsequent step and may then attach to their respective affinity reagent thereby forming the respective markers within the biological sample.

The marker and the method have the same advantages as the label. Further, the marker and the method may be supplemented with the features of the label described in this document, in particular, the features of the dependent claims of the label.

### Short Description of the Figures

Hereinafter, specific embodiments are described referring to the drawings, wherein:
- Figure 1: is a schematic view of a label comprising a nucleic acid backbone with a triplex structure and a plurality of labelling moieties,
- Figure 2: is a schematic view of a label without auxiliary and third oligonucleotides,
- Figure 3: is a schematic view of a label with a triplex structure according to another embodiment,
- Figure 4: is a schematic view of a label according to a second embodiment,

- Figure 5: is a schematic view of a marker comprising a hairpin loop label,
- Figure 6: is a schematic view a marker comprising a hairpin loop label according to a second embodiment,
- Figure 7: is a schematic view of circular labels,
- Figure 8: is a schematic view of a label according to a third embodiment,
- Figure 9: is a schematic view of a label according to a fourth embodiment,
- Figure 10: is a schematic view of a label according to a fifth embodiment and steps to generate the label,
- Figure 11: is a schematic view of a label according to a sixth embodiment and steps to generate the label,
- Figure 12: is a schematic view of a complex of the label according to Fig. 1 with a nucleic acid binding molecule, and
- Figure 13: is a schematic view of a complex of the label according to Fig. 1 with a further nucleic acid binding molecule.

### Detailed Description

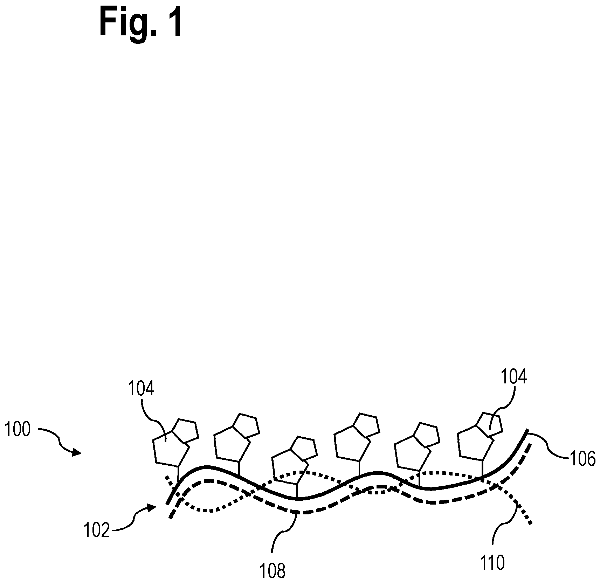

Figure 1 is a schematic view of a label 100 comprising a nucleic acid backbone 102 and a plurality of labelling moieties 104. The nucleic acid backbone 102 comprises a main oligonucleotide 106, an auxiliary oligonucleotide 108, and a third oligonucleotide 110. The labelling moieties 104 are attached to the main oligonucleotide 106, in particular covalently.

The main oligonucleotide 106 has a first nucleotide sequence and the auxiliary oligonucleotide 108 has a second nucleotide sequence. The first nucleotide sequence and the second nucleotide sequence are complementary to each other. Thus, the main oligonucleotide 106 and the auxiliary oligonucleotide 108 hybridise to each other along their entire length. This results in the nucleic acid backbone 102 having a duplex structure generated when the main oligonucleotide 106 and the auxiliary oligonucleotide 108 hybridise to each other. Thus, the main oligonucleotide 106 and the auxiliary oligonucleotide 108 form a double stranded nucleic acid molecule. In particular, the duplex structure of the nucleic acid backbone 102 may form a double helix from the main oligonucleotide 106 and the auxiliary oligonucleotide 108.

The third oligonucleotide 110 has a third nucleotide sequence, which is complementary to the duplex of the main oligonucleotide 106 and the auxiliary oligonucleotide 108, in particular based on Hoogsteen base-pairing. Thus, the main, auxiliary and third oligonucleotide 110 hybridise to each other to form a triplex structure.

Preferably, the nucleic acid backbone 102 comprises 20 to 260 nucleotides.

A corresponding marker may comprise the label 100 attached to an affinity reagent (not shown), such as an antibody, antibody fragment, aptamer, affibody, artificial polymeric binder, nanobody, or oligonucleotide probes. By means of the affinity reagent, the label 100 may specifically bind to a target analyte in a biological sample.

The labelling moieties 104 may be fluorophores like fluorescent proteins, or organic fluorescent dyes, QDots, Pdots, polymer dyes, SMILEs. The labelling moieties 104 may be for example fluorescent dyes such as ATTO 390, ATTO 425, ATTO 430, ATTO 465, ATTO 488, ATTO 532, ATOT 542, ATTO Rho3B, ATTO 565, ATTO 594, ATTO 643, ATTO 647N, ATTO 680, ATTO 700, ATTO 740, BODIPY dyes, AlexFluor^{™} dyes, Cy dyes, CF dyes. Such fluorescent dyes may be derivatives of a variety of base chromophore structures including but not limited to acridine, anthracenes, arylmethine, dipyrromethene, fluorescein, cyanines, coumarines, pyrene, oxazines, rhodamines, carborhodamines, squaraine, squaraine rotaxane, tetrapyrrole, triangelium dyes, xanthenes. Further labelling moieties 104 may be fluorescent proteins like GFP, mCherry, EYFP, Phycoerythrin, Alllophycocyanin or non-protein organic fluorophores like the aforementioned dyes or polymer dyes like SuperNova vn428 for example. Further, the labelling moieties 104 may be fluorescence quenchers like ATTO 540Q, ATTO 575Q, ATTO 580Q, ATTO 612Q. Further, the labelling moieties 104 may comprise fluorescent dyes that fluoresce only under a first condition but are essentially non-fluorescent under a second condition like for example ATTO MB2. Further, the labelling moieties 104 in the sense of this document may be a tandem dye, i.e. to covalently connected fluorophores.

The label 100, in particular its labelling moieties 104, and the marker comprising the label 100 may be optically detected, for example, by means of a microscope.

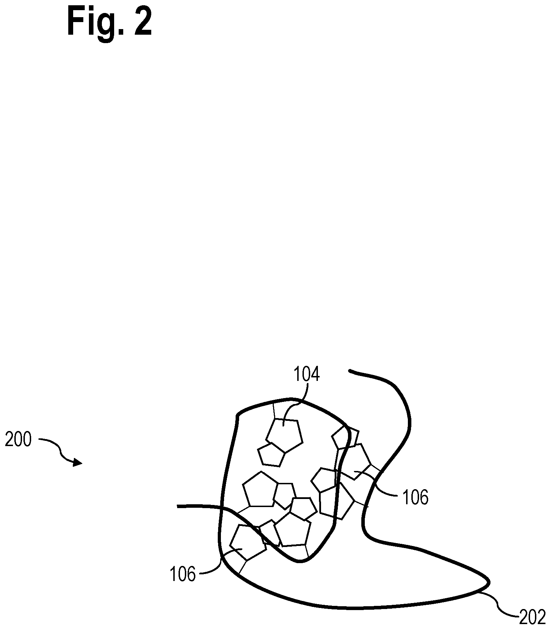

Figure 2 is a schematic view of a label 200 without the auxiliary oligonucleotide 108, in particular, without the second nucleotide sequence, and without the third oligonucleotide 110. In comparison to the label 100, the label 200 has a substantially more flexible nucleic acid backbone 202, consisting of one single stranded oligonucleotide. Specifically, the label 200 does not have a duplex or triplex structure.

The more flexible nucleic acid backbone 202 may result in the labelling moieties 104 aggregating, in particular when the labelling moieties 104 are hydrophobic and in an aqueous solution. This may result in a change in the optical properties of the labelling moieties 104, such as their absorbance wavelength or intensity. In particular, the labelling moieties 104 may shift their absorbance wavelength and/or exhibit self-quenching. This change in optical properties may complicate or prevent identification of the label 200 in a biological sample.

In contrast, the triplex structure of the nucleic acid backbone 102 enables the nucleic acid backbone 102 to be of significantly higher rigidity or stiffness. This results in the label 100 being substantially rod-shaped compared to the aggregated nature of the label 200. Moreover, this prevents the labelling moieties 104 attached to the nucleic acid backbone 102 from aggregating. In absence of aggregation, the labelling moieties 104 do not change their optical properties or exhibit their initial or inherent optical properties.

Figure 3 is a schematic view of a label 300 comprising the main oligonucleotide 106 and the auxiliary oligonucleotide 108, which are hybridised to each other to form a duplex structure. The labelling moieties 104 are attached to either the main or the auxiliary oligonucleotides 106, 108. A part of Figure 3 is based on a drawing found at https://commons.wikimedia.org/wiki/File:TriplexDNA(1BWG).png.

Additionally, the label 300 comprises three third oligonucleotides 302a, 302b, 302c which, together with the main oligonucleotide 106 and the auxiliary oligonucleotide 108, form a nucleic acid backbone 304 of the label 300. Each third oligonucleotide 302a, 302b, 302c may have a different third sequence that is specific to a particular segment or section of the duplex formed by the main and auxiliary oligonucleotides 106, 108 of the nucleic acid backbone 304. Thus, the nucleic acid backbone 304 comprises three triplex structures 306a, 306b, 306c formed by the respective third oligonucleotide 302a, 302b, 302c hybridising to the duplex of the main and auxiliary oligonucleotides 106, 108.

The triplex structures 306a, 306b, 306c of the nucleic acid backbone 304 enable the nucleic acid backbone 304 to be of significantly higher rigidity or stiffness compared to without the third oligonucleotides 302a, 302b, 302c. Compared to the aggregated nature of the label 200, this results in the label 300 being substantially rod-shaped. Moreover, this prevents the labelling moieties 104 attached to the nucleic acid backbone 304 from aggregating. In particular, any attractive forces between the labelling moieties 104 are countered by the rigidity of the nucleic acid backbone 304, in particular its triplex structure 306a, 306b, 306c. In absence of aggregation, the labelling moieties 104 do not change their optical properties or exhibit their initial or inherent optical properties.

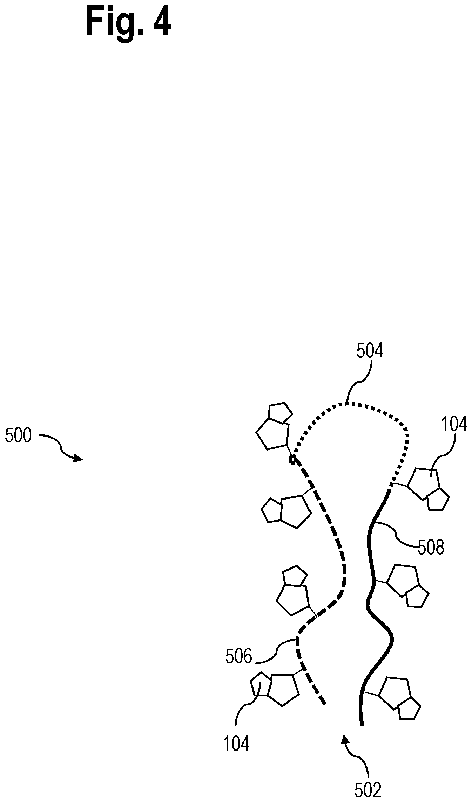

In the following, reference is made to the European patent application with the application number EP24177155.9, the complete content thereof being incorporated by reference herein, in particular Figures 3A to 3C and Figures 4A to 4C of EP24177155.9. In the description of these Figures, it is concluded that the flexibility of the ssDNA nucleic acid backbone is so high (in other words the persistence length is short) that the dye molecules can practically freely aggregate, despite the fact that they are connected to the polymeric backbone. It is proposed that a reduction of the flexibility or in other words an increase in persistence length or rigidity of the nucleic acid backbone, may reduce or eliminate aggregation and bring back the desired increase in label brightness with an increase in the number of dyes. This is confirmed by experiments, showing that the ssDNA nucleic backbone of the label was hybridized with one or more complementary oligonucleotide(s) to generate dsDNA nucleic acid backbones and a "rescue" of the ATTO 550 and ATTO 647N labels fluorescence emission by formation of a nucleic acid backbone comprising a duplex. Duplex formation increases the rigidity or persistence length of the nucleic acid backbone to the point where the connected dye molecules can no longer freely aggregate.

By providing a label with a nucleic acid backbone with a triplex structure, the rigidity of the nucleic acid backbone can be further increased. For example, the label 300 comprising the nucleic acid backbone 304 with the triplex structures 306a, 306b, 306c has an increased rigidity, in particular the nucleic acid backbone 304 has an increased persistence length. This is, in particular, in comparison to the nucleic acid backbone disclosed in the European patent application with the application number EP24177155.9.

Figure 4 is a schematic view of a label 500. The label 500 comprises a plurality of the labelling moieties 104. The label 500 further comprises a nucleic acid backbone 502 consisting of a single main oligonucleotide 504 with a first nucleotide sequence 506 and a second nucleotide sequence 508. The second nucleotide sequence 508 is the reverse complement of the first nucleotide sequence 506. Thus, the first nucleotide sequence 506 and the second nucleotide sequence 508 may hybridise to form a duplex structure. In Fig. 4 the first and second nucleotide sequences 506, 508 are shown in a non-hybridised state without the duplex structure. The nucleic acid backbone 502 may form a stem-loop upon hybridisation of the first and second nucleotide sequences 506, 508. The resulting stem or duplex structure of the nucleic acid backbone 502, comprising the first and second nucleotide sequences 506, 508, may, in particular, form a double helix structure. The label 500 further comprises a third oligonucleotide (not shown) that comprises a third nucleotide sequence, which is complementary to at least a part of the stem duplex structure formed by the first and second nucleotide sequences 506, 508. Hybridisation of the third oligonucleotide to the stem forms a triplex structure, which increases the rigidity of the nucleic acid backbone 502 of the label 500.

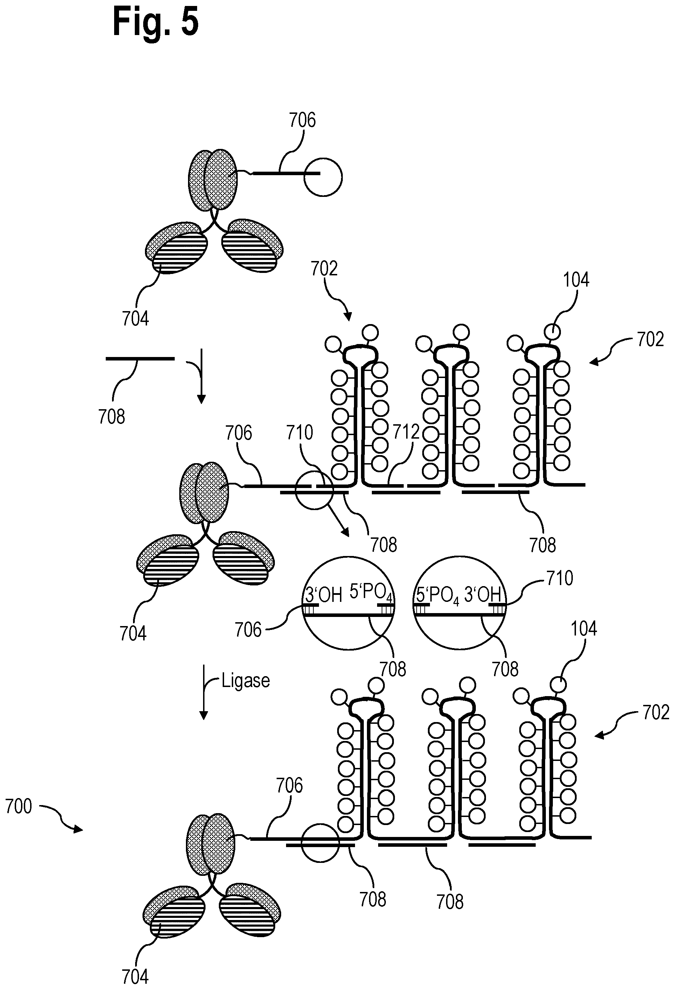

Figure 5 is a schematic view of a marker 700 comprising several hairpin loop labels 702, as well as steps to generate the marker 700. The marker 700 further comprises an affinity reagent 704 for specifically binding the marker 700 to a target analyte. The affinity reagent 704 may be an antibody, for example. Alternatively, the affinity reagent 704 may be an antibody fragment or an aptamer. The labels 702 may preferably have the features described for the label 500. In particular, the labels 702 each comprise a third oligonucleotide which forms a triplex structure (not explicitly shown).

The marker 700 may further comprise a barcode oligonucleotide 706 that is attached to the affinity reagent 704 and by means of which the labels 702 may be attached to the affinity reagent 704.

In order to generate the marker 700 from the affinity reagent 704 and the labels 702 an overlapping complementary attachment oligonucleotide 708 may be introduced to the parts of the marker 700 (middle view of Fig. 5). In particular, the labels 702 may be attached to each other and attached to the affinity reagent 704 by means of the attachment oligonucleotide 708. The attachment oligonucleotide 708 is at least partially complementary to the barcode oligonucleotide 706 and to a free end 710 of a main oligonucleotide of a nucleic acid backbone of the label 702. The attachment oligonucleotide 708 may also be complementary to a second free end 712 of the main oligonucleotide of the label 702. This enables attaching the labels 702 to each other.

In an alternative embodiment, a plurality of attachment oligonucleotides 708 may be introduced to the parts of the marker 700. Further, the barcode oligonucleotide 706 and the free ends 710, 712 of each label 702 may have a unique sequence and at least one of the attachment oligonucleotides 708 may have a sequence complementary to the respective adjacent sequences of the barcode oligonucleotide 706 and free ends 710, 712. This enables specifically attaching the individual labels 702 to the marker 700.

In a subsequent step, the labels 702, in particular their nucleic acid backbones, may be ligated to each other and to the attachment oligonucleotide 706 (bottom view of Fig. 5) by adding a ligase. This catalyses the formation of a phosphodiester bond between the 3'-hydroxyl group (-OH) at one end of one of the nucleotide strands 706, 710, 712 and the 5'-phosphate group (-PO₄) of another end of the respective nucleotide strands 706, 710, 712. This results in the robust marker 700.

In an alternative embodiment the marker 700 may comprise only a single label 702.

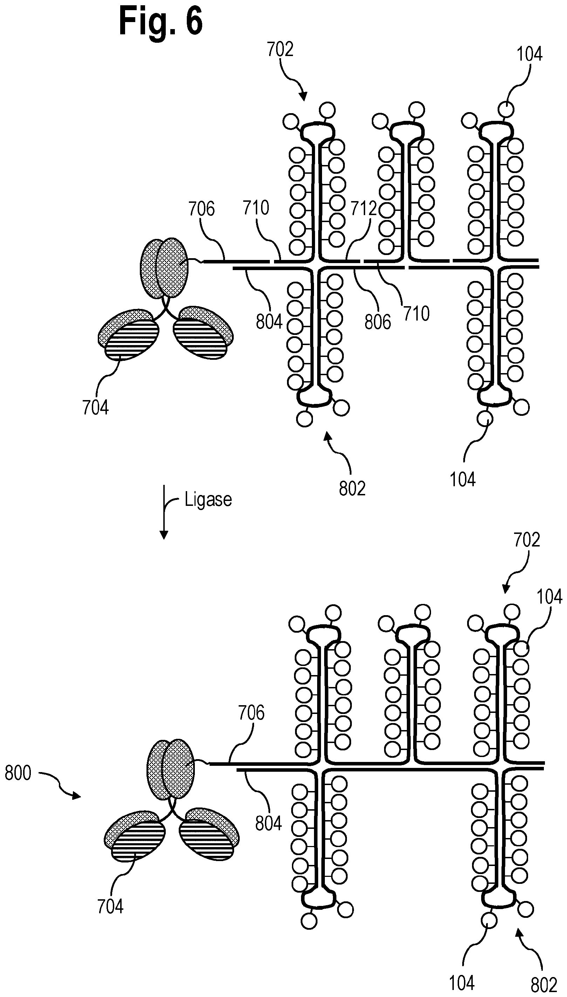

Figure 6 is a schematic view of a marker 800 comprising hairpin loop labels 702, 802, as well as a step to generate the marker 800. The marker 800 further comprises the affinity reagent 704 with the barcode oligonucleotide 706. The labels 802 may preferably have the features described for the label 500.

Similarly to the marker 700 described together with Fig. 5, the label 800 comprises the label 702. However, instead of attaching the label 702 to the barcode oligonucleotide 706 by means of the attachment oligonucleotide 708, the label 702 is attached to the barcode oligonucleotide 706 by means of the label 802 to generate the marker 800. In particular, the label 802 comprises a complementary barcode oligonucleotide 804, which is at least partially complementary to the barcode oligonucleotide 706 of the affinity reagent 704 and to the free end 710 of the label 702. The complementary barcode oligonucleotide 804 is preferably a single stranded part of the nucleic acid backbone of the label 802.

The label 802, in particular its main oligonucleotide, may have a further end 806, which is at least partially complementary to the free ends 710, 712 of adjacent labels 702. The parts 804, 806 of the label 802 may alternatively both be at least partially complementary to the free ends 710, 712 of respective adjacent labels 702 in order to connect a plurality of labels 702 to each other.

As described for marker 700, in a subsequent step, the adjacent labels 702, 802, in particular their nucleic acid backbones, may be ligated to the respective adjacent labels 702, 802 and to the attachment oligonucleotide 706 (bottom view of Fig. 6) by adding a ligase. This catalyses the formation of a phosphodiester bond between the 3'-hydroxyl group (-OH) at one end of one of the nucleotide strands 706, 710, 712, 804, 806 and the 5'-phosphate group (-PO₄) of another end of the respective nucleotide strands 706, 710, 712, 804, 806. This results in the robust marker 800.

In view of the duplex structure of the nucleic acid backbones of the labels 702, 802 and the duplex structure that is generated from hybridising the oligonucleotides 706, 710, 712 to the attachment oligonucleotide 708 or the oligonucleotides 804, 806, the markers 700, 800 have a rigid structure. This enables keeping the labelling moieties 702 in place relative to each other and avoiding their aggregation.

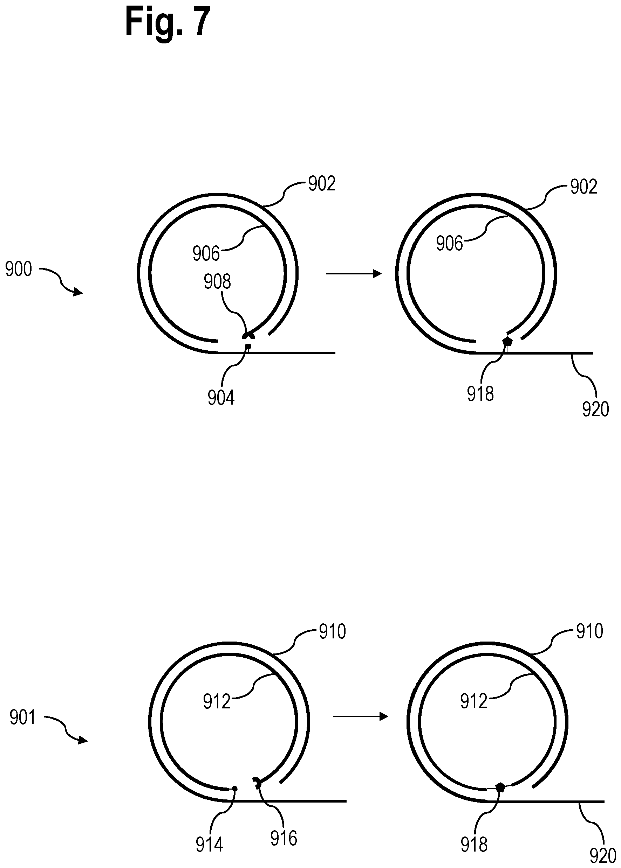

Figure 7 is a schematic view of circular labels 900, 901. The label 900 comprises a main oligonucleotide 902 with a first reaction moiety 904 and an auxiliary oligonucleotide 906 with a second reaction moiety 908. In addition, the label 900 comprises a third oligonucleotide (not shown) which hybridises to the main oligonucleotide 902 and the auxiliary oligonucleotide 906 forming a triplex. The main oligonucleotide 902 comprises a first nucleotide sequence that is complementary to a second nucleotide sequence of the auxiliary oligonucleotide 906. The main oligonucleotide 902 and the auxiliary oligonucleotide 906 may therefore hybridise and form a duplex structure, in particular a double helix. The first reaction moiety 904 and the second reaction moiety 908 are both arranged towards a 3' or a 5' end of the respective oligonucleotide 902, 906.

The label 901 similarly comprises a main oligonucleotide 910 and an auxiliary oligonucleotide 912, which comprise respective complementary first and second nucleotide sequences that may form a duplex structure. In addition, the label 901 comprises a third oligonucleotide (not shown) which hybridises to the main oligonucleotide 910 and the auxiliary oligonucleotide 912 forming a triplex. The auxiliary oligonucleotide 912 comprises a first reaction moiety 914 towards a first end, for example at a 3' or a 5' end, of the auxiliary oligonucleotide 912. In addition, the auxiliary oligonucleotide 912 comprises a second reaction moiety 916 towards a second end, for example a 5' or a 3' end, of the auxiliary oligonucleotide 912, opposing the first end.

The first reaction moieties 904, 914 and the second reaction moiety 908, 916 are configured to form a covalent bond 918 with each other, in particular under specific reaction conditions or in the presence of a particular catalyst. This cross-links the respective parts of the labels 900, 901. For example, the first reaction moieties 904, 914 and the second reaction moieties 908, 916 may be respective click chemistry groups, in particular based on copper-catalysed azide-alkyne cycloaddition.

The first reaction moieties 904, 914 and the second reaction moiety 908, 916 are preferably not attached along the first or second nucleotide sequence of the respective oligonucleotide 902, 906, 910, 912. Instead, the first reaction moieties 904, 914 and the second reaction moiety 908, 916 are preferably attached to the respective oligonucleotide 902, 906, 910, 912 next to the first or second nucleotide sequence.

The main oligonucleotides 902, 910 of the labels 900, 901 may preferably comprise a single stranded free end 920 that is complementary to a barcode oligonucleotide of an affinity reagent, in order to attach the labels 900, 901 to the affinity reagent. The free end 920 does not comprise the first nucleotide sequence of the main oligonucleotide 902, 910. In case of the label 900, the first reaction moiety 904 is preferably arranged at a distance from a 3' or 5' end of the main oligonucleotide 902 in order to generate the free end 920.

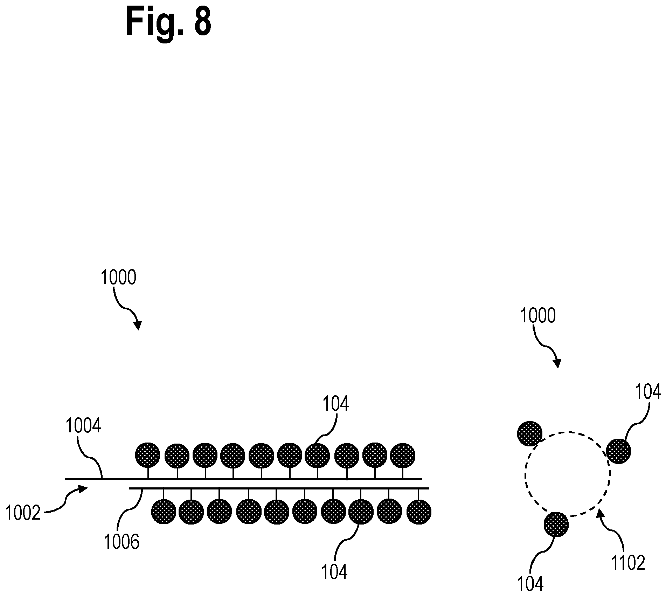

Figure 8 is a schematic view of a label 1000. The label 1000 comprises a nucleic acid backbone 1002 with a main oligonucleotide 1004 and an auxiliary oligonucleotide 1006. In addition, the nucleic acid backbone 1002 comprises a third oligonucleotide (not shown) which hybridises to the main oligonucleotide 1004 and the auxiliary oligonucleotide 1006 forming a triplex. A plurality of labelling moieties 104 is attached to a triplex structure formed between the main oligonucleotide 1004, the auxiliary oligonucleotide 1006, and the third oligonucleotide. The right-hand view of Fig. 8 shows the label 1000 along its longitudinal axis and schematically shows the nucleic acid backbone 1002.

The labelling moieties 104 are attached to the main and auxiliary oligonucleotides 1004, 1006 such that they are evenly spaced around the duplex structure of the nucleic acid backbone 1002. For example, the evenly spaced attachment is achieved by selecting appropriate sequences for the main and auxiliary oligonucleotides 1004, 1006, which allow the controlled formation of the duplex.

In addition, or as alternative to specific sequences the main and auxiliary oligonucleotides 1004, 1006 may also comprise repetitive elements and/or may comprise poly(T), poly(A), poly(C), poly(G) stretches. In one preferred embodiment, the main oligonucleotide 1004 comprises poly(T) sequences, which can be manufactured in a particularly cost-effective manner. For example, a label may comprise poly(T) main oligonucleotide and the auxiliary oligonucleotide(s) may be one or multiple poly(A) oligonucleotides.

In another particularly preferred embodiment, the main and auxiliary oligonucleotides 1004, 1006 comprise specific sequences that allow the controlled hybridization, which enables precise control over labelling moieties 104 that are attached to both the main and auxiliary oligonucleotide. As a dsDNA helix makes a full-turn every 10nt, which corresponds roughly to 3.4 nm (referred to as the pitch), the attachment positions along the auxiliary and main oligonucleotides 1004, 1006 may be selected such that the positioning of labelling moieties 104 on the duplex is optimized with respect to dye-to-dye distance and orientation in space. This is schematically depicted in the right-hand view of Fig. 8.

In a particularly preferred embodiment, the dyes are conjugated to nucleobases to opposing nucleobases of the main and auxiliary oligonucleotides 1004, 1006 and the dye-to-dye distance along the main and auxiliary oligonucleotides 1004, 1006 is in the range of 3 nt to 30 nt, more preferably 3 nt to 20 nt.

In a particularly preferred embodiment, the main oligonucleotide 1004 of the label 1000 is attached to a first labelling moiety and the auxiliary oligonucleotide 1006 is attached to a second labelling moiety, wherein in the first and second labelling moieties comprise, or are, a FRET pair such as for example the following pairs of ATTO dyes:
FRET Donor - FRET Acceptor(s)
ATTO 425 - ATTO 520
ATTO 488 - ATTO 550, ATTO 565, ATTO 647**N**, ATTO 655
ATTO 520 - ATTO 647**N**
ATTO 532 - ATTO 647**N**, ATTO 655
ATTO 550 - ATTO 590, ATTO 647**N**
ATTO 565 - ATTO 590, ATTO 647**N**
ATTO 590 - ATTO 620, ATTO 647**N**, ATTO 680
ATTO 620 - ATTO 680

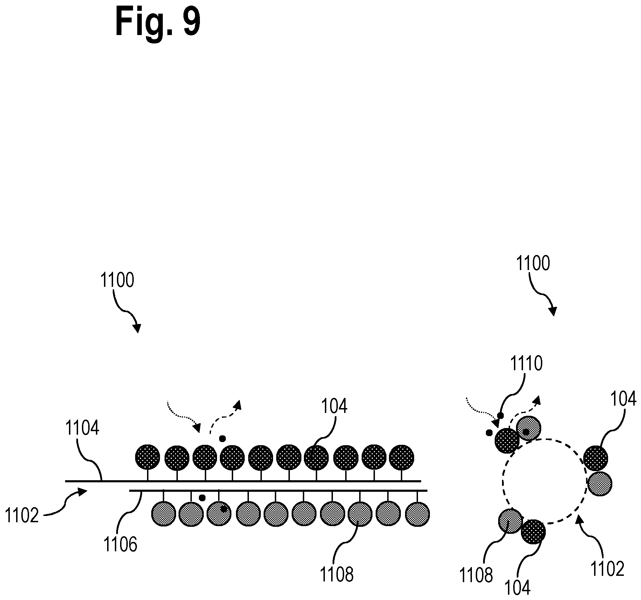

Figure 9 is a schematic view of a label 1100. The label 1100 comprises a nucleic acid backbone 1102 with a main oligonucleotide 1104, an auxiliary oligonucleotide 1106, and a third oligonucleotide (not shown). The main oligonucleotide 1104, the auxiliary oligonucleotide 1106, and the third oligonucleotide form a triplex structure. A plurality of labelling moieties 104 is attached to the main oligonucleotide 1004, whereas a plurality of reactive oxygen species quenchers 1108 is attached to the auxiliary oligonucleotide 1006. The right-hand view of Fig. 9 shows the label 1100 along its longitudinal axis and schematically shows the nucleic acid backbone 1102.

The reactive oxygen species quenchers 1108 are configured to reduce the occurrence or concentration of reactive oxygen species 1110 in their proximity. This enables an increase in the lifetime of the labelling moieties 104. Examples of the reactive oxygen species quenchers 1108 include DABCO and lycopene.

The labelling moieties 104 and the reactive oxygen species quenchers 1108 are attached to the respective main and auxiliary oligonucleotides 1104, 1106 such that each of the reactive oxygen species quenchers 1108 is in close proximity to one of the labelling moieties 104. Preferably, the reactive oxygen species quenchers may be attached to the nucleic acid backbone at a distance in a range of 0.3 nm to 15 nm to one of the labelling moieties.

A preferred arrangement for two labelling moieties such as a first labelling moiety 104 (e.g. dye) that shall be brought into close proximity to a second labelling moiety 1108 (e.g. ROS quencher or a FRET partner donor or acceptor) via the duplex formation between the main and auxiliary oligonucleotide 1004, 1006 is to place the first and second labelling moiety 104, 1108 at a distance of 3 to 6 nt along the duplex. On a natural dsDNA duplex 5 nt corresponds to a half helix turn, which means that if the first labelling moiety 104 is at position 1 on the main oligonucleotide 1104 and the second labelling moiety 1108 is placed at position 5 or 6 of the auxiliary oligonucleotide 1106 than the first and second labelling moieties 104, 1108 are on the same side of the helix and about 1.7 nm apart. In this case the positions are counted from the same end of the helix, i.e. base pairs share the same position count. In light of the fact that typical R0 values (Förster radii) for FRET between ATTO dye pairs (2-7 nm) as well as other organic fluorescent dyes, Qdots, Pdots, fluorescent proteins are typically in the range of 1-10 nm, this allows the effective generation of FRET-based labels. The attachment of FRET donor and acceptor to different, i.e. the main and auxiliary oligonucleotides 1104, 1106, is particular advantageous as it allows the effective generation of FRET-based labels from a library of conjugated main and auxiliary oligonucleotides 1104, 1106.

Further, the labelling moieties 104 and the reactive oxygen species quenchers 1108 may preferably be evenly spaced around the triplex structure of the nucleic acid backbone 1102.

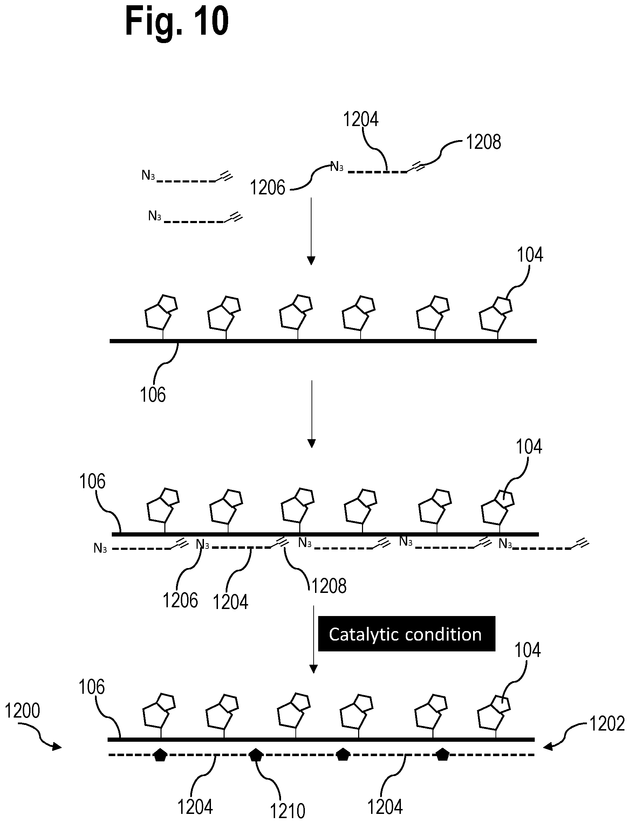

Figure 10 is a schematic view of a label 1200 and steps to generate the label 1200. The label 1200 comprises a nucleic acid backbone 1202 with the main oligonucleotide 106, to which labelling moieties 104 are attached. The nucleic acid backbone 1202 further comprises a plurality of auxiliary oligonucleotides 1204 hybridised to the main oligonucleotide 106. In order to hybridise the auxiliary oligonucleotides 1204 to the main oligonucleotide 106, the main oligonucleotide 106 may comprise a single continuous first nucleotide sequence and each of the auxiliary oligonucleotides 1204 may have a second sequence that is complementary to unique or repetitive, identical parts of the first nucleotide sequence of the main oligonucleotide 106. Alternatively, the main oligonucleotide 106 may comprise several unique or identical first nucleotide sequences that at least some of the auxiliary oligonucleotides 1204 are complementary to.

In addition, the auxiliary oligonucleotides 1204 comprise a first reaction moiety 1206 and a second reaction moiety 1208. In particular, the first reaction moiety 1206 is attached to one of a 3' and 5' end of the auxiliary oligonucleotide 1208 and the second reaction moiety 1208 is attached to the other one of the 3' and 5' end of the auxiliary oligonucleotide 1204.

The first reaction moiety 1206 and the second reaction moiety 1208 are configured to form a covalent bond 1210 with each other, in particular under specific catalytic conditions or in the presence of a particular catalyst. This cross-links the auxiliary oligonucleotides 1204 of the label 1200. For example, the first reaction moiety 1206 and the second reaction moiety 1208 may be respective click chemistry groups, in particular based on copper-catalysed azide-alkyne cycloaddition. This forms a triazole linkage between the auxiliary oligonucleotide 1204. In an alternative embodiment, the first reaction moiety 1206 and the second reaction moiety 1208 are configured to form a squaramide linkage. Generally, the covalent bond 1210 increases the stiffness of the nucleic acid backbone 1202 compared to phosphodiester bonds of natural nucleic acids.

The label 1200 may be generated by initially contacting main oligonucleotide 106 with the auxiliary oligonucleotides 1204 in order to hybridise the main oligonucleotide 106 with the auxiliary oligonucleotides 1204. This generates the duplex structure of the nucleic acid backbone 1202.

In a subsequent step, a particular catalytic condition may be applied to the auxiliary oligonucleotides 1204, such as the addition of a catalyst. This forms the covalent bond 1210 between the auxiliary oligonucleotides 1204.

The label 1200 further comprises a third oligonucleotide (not shown), which may be hybridised to the main and auxiliary oligonucleotides 106, 1204 such that they form a triplex structure. The third oligonucleotide may be added after forming the covalent bond 1210.

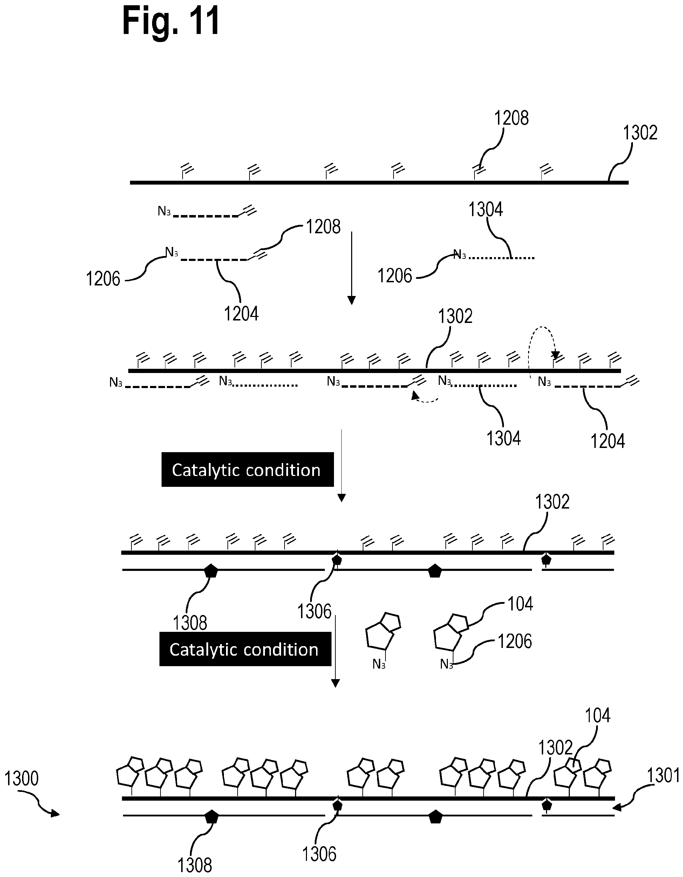

Figure 11 is a schematic view of a label 1300 and steps to generate the label 1300. The label 1300 comprises a nucleic acid backbone 1301 with a main oligonucleotide 1302 and a plurality of auxiliary oligonucleotides 1204, 1304. The main oligonucleotide 1302 comprises a first nucleotide sequence that the second nucleotide sequence of each auxiliary oligonucleotide 1204, 1304 are complementary to. In particular, the auxiliary oligonucleotides 1204 may have a different second nucleotide sequence than the auxiliary oligonucleotide 1304. This enables generating the label 1300 with a particular order of the auxiliary oligonucleotides 1204, 1304 along the duplex structure of the nucleic acid backbone 1301.

The auxiliary oligonucleotides 1204 comprise the first reaction moiety 1206 and the second reaction moiety 1208. The auxiliary oligonucleotides 1304 comprise only the first reaction moiety 1206, preferably at a 3' or 5' end. The main oligonucleotide 1302 comprises several second reaction moieties 1208.

The second reaction moieties 1208 are arranged along the main oligonucleotide 1302 such that they are at a distance from the first reaction moieties 1206, when the second nucleotide sequence of the auxiliary oligonucleotides 1304 are hybridised to the first nucleotide sequence of the main oligonucleotide 1302, that respective first and second reaction moieties 1206, 1208 can form a covalent bond 1306.

The first reaction moieties 1206 of the auxiliary oligonucleotides 1304 may form a covalent bond 1308 with second reaction moieties 1208 of adjacent auxiliary oligonucleotides 1204.

Generally, the covalent bonds 1306, 1308 cross-link the nucleic acid backbone 1301 and increase the stiffness of the nucleic acid backbone 1301 compared to base-pairing interactions or phosphodiester bonds of natural nucleic acids.

Optionally, the labelling moieties 104 may similarly comprise first reaction moieties 1206 and be attached to respective second reaction moieties 1208 of the main oligonucleotide 1302.

The label 1300 may be generated by initially hybridising the auxiliary oligonucleotides 1304, 1204 to the main oligonucleotide 1302. Subsequently, the auxiliary oligonucleotides 1304, 1204 and the main oligonucleotide 1302 may be cross-linked by applying a catalytic condition to form covalent bonds 1306, 1308 between the auxiliary oligonucleotides 1304, 1204 and the main oligonucleotide 1302.

In a subsequent step, the labelling moieties 104 with first reaction moieties 1206 may be brought into contact with the main oligonucleotide 1302 and the labelling moieties 104 may be attached to the main oligonucleotide 1302 by applying a respective catalytic condition.

In a particular embodiment, the first and second reaction moieties 1206, 1208 for attaching the labelling moieties 104 to the main oligonucleotide 1302 may be different to the first and second reaction moieties 1206, 1208 for cross-linking the auxiliary oligonucleotides 1304, 1204 and the main oligonucleotide 1302.

The label 1300 further comprises a third oligonucleotide (not shown), which may be hybridised to the main and auxiliary oligonucleotides 1302, 1204, 1304 such that they form a triplex structure. The third oligonucleotide may be added after forming the covalent bonds 1306, 1308.

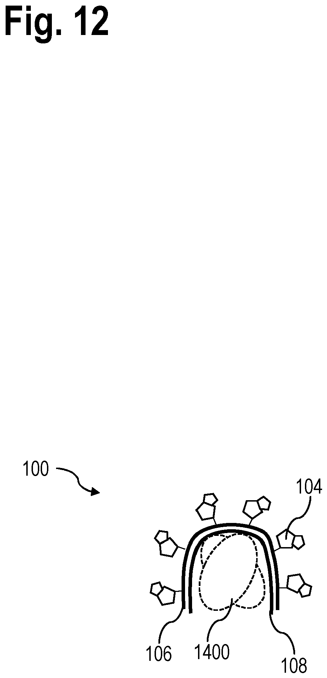

Figure 12 is a schematic view of a complex of the label 100 with a nucleic acid binding molecule 1400. The nucleic acid backbone 102 of the label 100 may be bound to the nucleic acid binding molecule 1400 in order to bend the backbone 102 and increase the stiffness or rigidity of the nucleic acid backbone 102. For clarity, the third oligonucleotide 110 is omitted from the Fig. 12.

The nucleic acid binding molecule 1400 may be a transcription factor (e.g. bHLH), a histone, or prokaryotic histones such as IHF or HU. Some nucleic acid binding molecules may bind nucleic acid duplex and/or triplex structures in a sequence-specific manner, others in an unspecific manner. Depending on the particular nucleic acid binding molecule 1400, the nucleic acid backbone 102, in particular its duplex and/or triplex structures, may comprise a particular recognition sequence.

An example of a nucleic acid binding molecule is disclosed by Rice et al. Cell, Vol. 87, 1295-1306, December 27, 1996 (Crystal Structure of an IHF-DNA Complex: A Protein-Induced DNA U-Turn). Using IHF as a nucleic acid binding molecule may bend the nucleic acid backbone 102 into one or a plurality of U-turns. This allows for label compaction and labelling moiety placing on the backbone 102 in a way that achieves optimal spatial separation of the labelling moieties 104.

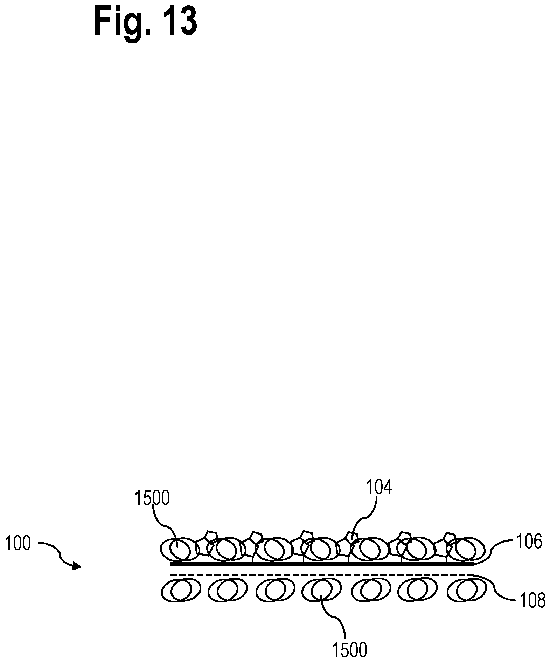

Figure 13 is schematic view of a complex of the label 100 with a nucleic acid binding molecule 1500. The nucleic acid backbone 102 of the label 100 may be bound to the nucleic acid binding molecule 1500 in order to increase the stiffness or rigidity of the nucleic acid backbone 102. For clarity, the third oligonucleotide 110 is omitted from the Fig. 13.

Suitable nucleic acid binding molecules may include histones. Histones with unconventional DNA-binding modes in vitro are major chromatin constituents in the bacterium Bdellovibrio bacteriovorus (Hocher et al., Nature Microbiology, Volume 8, November 2023, 2006-2019).

Further to the nucleic acid binding molecules described for Figures 12 and 13, a nucleic acid binding molecules may be provided that bind to the triplex structure. For example, the stability of triplexes may be strongly enhanced by adding intercalators, i.e. small molecules that stabilize the triplex by intercalation such as 3,3#-diethlyoxadicarbocyanine iodide, BePI, acridine, proflavine, BQQ, coralyne chloride, N-[2,2-(diethylamino)ethyl]-2-naphthyl-quinoline-4-amine, 2,6-amidoanthaquinones, ethidium bromide as described in Pozza et al. Three's a crowd - stabilisation, structure, and applications of DNA triplexes Chem. Sci., 2022, 13, 10193-10215.

Similarly, triplex stability may be enhanced by DNA triplex groove binders, which include but are not limited to DAPI, Hoechst33258, Hoechst 33342, Berenil, Distamycin, as described in Pozza et al. Three's a crowd - stabilisation, structure, and applications of DNA triplexes Chem. Sci., 2022, 13, 10193-10215.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

**Reference signs**

| | |
|---|---|
| 100, 200, 300, 500, 702, 802, 900, 901, 1000, 1200, 1300 | Label |
| 102, 202, 304, 502, 1002, 1102, 1202, 1301 Nucleic | acid backbone |
| 104 | Labelling moiety |
| 106, 504, 902, 910, 1004, 1104, 1302 | Main oligonucleotide |
| 108, 906, 912, 1006, 1106, 1204, 1304 | Auxiliary oligonucleotide |
| 110, 302a, 302b, 302c | Third oligonucleotide |
| 202 | Flexible nucleic acid backbone |
| 306a, 306b, 306c | Triplex structure |
| 506 | First nucleotide sequence |
| 508 | Second nucleotide sequence |
| 600 | Double helix |
| 700,800 | Marker |
| 704 | Affinity reagent |
| 706 | Barcode oligonucleotide |
| 708 | Attachment oligonucleotide |
| 710 | Free end of main oligonucleotide |
| 712 | Second free end of main oligonucleotide |
| 804 | Complementary barcode oligonucleotide |
| 806 | Further end of main oligonucleotide |
| 904, 914, 1206 | First reaction moiety |
| 908, 916, 1208 | Second reaction moiety |
| 918, 1210, 1306, 1308 | Covalent bond |
| 920 | Free end of main oligonucleotide |
| 1108 | Reactive oxygen species quencher |
| 1110 | Reactive oxygen species |
| 1400, 1500 | Nucleic acid binding molecule |

## Claims

1. A label (100, 300, 500, 702, 802, 900, 901, 1000, 1200, 1300) for analysing a biological sample comprising
a nucleic acid backbone (102, 304, 502, 1002, 1102, 1202, 1301),
a first plurality of labelling moieties (104), and
wherein the nucleic acid backbone (102, 304, 502, 1002, 1102, 1202, 1301) comprises a triplex structure (306a, 306b, 306c).

2. The label according to claim 1, wherein the nucleic acid backbone (102, 304, 502, 1002, 1102, 1202, 1301) comprises a main oligonucleotide (106, 504, 902, 910, 1004, 1104, 1302) with at least a first nucleotide sequence, wherein the nucleic acid backbone (102, 304, 502, 1002, 1102, 1202, 1301) comprises at least a second nucleotide sequence, and wherein at least the first nucleotide sequence and the second nucleotide sequence form a duplex structure.

3. The label according to claim 2, wherein the first nucleotide sequence and the second nucleotide sequence are at least partially complementary to each other.

4. The label according to one of the preceding claims 2 to 3, wherein the nucleic acid backbone (102, 304, 502, 1002, 1102, 1202, 1301) comprises at least a third oligonucleotide (110, 302a, 302b, 302c) with a third nucleotide sequence, and wherein the third nucleotide sequence forms the triplex structure (306a, 306b, 306c) with the first nucleotide sequence and the second nucleotide sequence.

5. The label according to claim 4, wherein the third nucleotide sequence is at least partially complementary to the hybridised first nucleotide sequence and second nucleotide sequence.

6. The label according to one of the preceding claims 4 to 5, wherein the nucleic acid backbone (102, 304, 502, 1002, 1102, 1202, 1301) comprises several third oligonucleotides (110, 302a, 302b, 302c).

7. The label according to one of the preceding claims 2 to 6, wherein the main oligonucleotide (106, 504, 902, 910, 1004, 1104, 1302) further comprises the second nucleotide sequence, and wherein the second nucleotide sequence is a reverse complement of the first nucleotide sequence.

8. The label according to claim 7, wherein the nucleic acid backbone (102, 304, 502, 1002, 1102, 1202, 1301) comprises several of the main oligonucleotide (106, 504, 902, 910, 1004, 1104, 1302).

9. The label according to one of the preceding claims 2 to 5, wherein the nucleic acid backbone (102, 304, 502, 1002, 1102, 1202, 1301) comprises at least one auxiliary oligonucleotide (108, 906, 912, 1006, 1106, 1204, 1304) comprising the second nucleotide sequence.

10. The label according to one of the preceding claims 2 to 9, wherein the nucleic acid backbone (102, 304, 502, 1002, 1102, 1202, 1301) comprises a plurality of the first nucleotide sequence.

11. The label according to one of the preceding claims, comprising at least one nucleic acid binding molecule.

12. The label according to one of the preceding claims, wherein the labelling moieties (104) are hydrophobic labelling moieties.

13. The label according to one of the preceding claims, wherein the labelling moieties (104) are attached to the nucleic acid backbone (102, 304, 502, 1002, 1102, 1202, 1301) at a distance from each other in a range from one to ten nucleotides.

14. The label according to one of the preceding claims, wherein the labelling moieties (104) are attached to the main oligonucleotide (106, 504, 902, 910, 1004, 1104, 1302).

15. The label according to one of the preceding claims, wherein the auxiliary oligonucleotide (108, 906, 912, 1006, 1106, 1204, 1304) or the third oligonucleotide (110, 302a, 302b, 302c) comprises at least a second plurality of labelling moieties configured to excitonically interact with the first plurality of labelling moieties (104).

16. The label according to one of the preceding claims, wherein the nucleic acid backbone (102, 304, 502, 1002, 1102, 1202, 1301) comprises 20 to 260 nucleotides, and/or wherein the main oligonucleotide (106, 504, 902, 910, 1004, 1104, 1302) has a length of 20 to 120 nucleotides.

17. The label according to one of the preceding claims, wherein the nucleic acid backbone (102, 304, 502, 1002, 1102, 1202, 1301) comprises a barcode sequence.

18. The label according to any of the preceding claims comprising at least 5 labelling moieties (104).

19. A marker for analysing a biological sample comprising:
a label (100, 300, 500, 702, 802, 900, 901, 1000, 1200, 1300) according to one of the preceding claims, and
an affinity reagent configured to specifically bind to a target analyte.

20. The marker according to claim 19, wherein the affinity reagent comprises a barcode sequence.

21. A method for analysing a biological sample comprising the steps:
introducing into the biological sample at least one marker or parts of at least one marker according to one of the preceding claims 19 and 20, and
generating a readout of the biological sample with the marker.
